Europäisches Patentamt

European Patent Office

Office européen des brevets

(19)

(11) **EP 0 463 140 B1**

(12) **EUROPEAN PATENT SPECIFICATION**

(45) Date of publication and mention
of the grant of the patent:
**01.04.1998 Bulletin 1998/14**

(21) Application number: **91902188.1**

(22) Date of filing: **15.01.1991**

(51) Int Cl.⁶: **C12N 9/08**, C12N 5/04

(86) International application number:
**PCT/GB91/00051**

(87) International publication number:
**WO 91/10729 (25.07.1991 Gazette 1991/17)**

(54) **production of a peroxidase**

herstellung einer peroxidase

production d'une peroxydase

(84) Designated Contracting States:
**CH DE DK FR GB IT LI NL SE**

(30) Priority: **15.01.1990 GB 9000827**

(43) Date of publication of application:
**02.01.1992 Bulletin 1992/01**

(73) Proprietor: **PHYTERA, INC.
Worcester, Massachusetts 01605 (US)**

(72) Inventors:
• **FOWLER, Michael, William Oker End Stich Lane
Oker
Derbyshire DE4 2JP (GB)**
• **STEPAN-SARKISSIAN, Gagik
Sheffield S10 5BX (GB)**
• **GREY, Debbie 71 Ashgate Avenue
Chesterfield S40 1JD (GB)**

(74) Representative: **Harding, Charles Thomas et al
D. Young & Co.
21 New Fetter Lane
London EC4A 1DA (GB)**

(56) References cited:
• **J. Chem. Tech. Biotechnol., vol. 38, 1987, Society
of Chemical Industry, (GB); Y. Yamada et al.:
"Production of horse radish peroxidase by plant
cell culture", pages 31-39**
• **Physiologia Plantarum, vol. 23, 1970; L.K.
Simola et al.: "Changes in the activity of certain
enzymes of Acer pseudoplatanus L. cells at four
stages of growth in suspension culture", pages
1212-1222**
• **Patent Abstracts of Japan, vol. 4, no. 546 (C-661),
12 June 1989; & JP-A-01222778 (NITTO DENKO
CORP.) 6 September 1989**
• **Plant Science, vol. 42, 1985, Elsevier Scientific
Publishers Ireland Ltd, (IE); A. Drotar et al.:
"Evidence for glutathione peroxidase activities
in cultured plant cells", pages 35-40**
• **Experimental Cell Research, vol. 33, 1964; D.T.A.
Lamport: "Cell suspension cultures of higher
plants: isolation and growth energetics", pages
195-206**

**Description**

This invention relates to a process for the production of peroxidase enzymes from plant cell cultures.

Peroxidase enzymes catalyse a host of reactions in which hydrogen peroxide is a specific oxidising agent and a wide range of substrates act as electron donors. It is widely used at the moment in diagnostic kits, and among other things it has potential use in other industries such as paper recycling, chemical, waste water treatment, and in detergents and bleaching agents.

Peroxidase enzymes are widely distributed in nature and are produced by a wide variety of plant species. At the present time, however, the chief commercial source is horseradish. In the commercial production of horseradish peroxidase (HRP) the horseradish roots are harvested and the sprouted roots are minced mechanically in water to form a starting material. From this, peroxidase is purified by a series of ammonium sulphate and ethanol precipitations. To obtain high purity enzyme, conventional chromatographic techniques are employed. Unfortunately, this process leads to large quantities of waste tissue and problems arise with dispersing of waste. Not only this but there is a significant irreversible loss of enzyme activity resulting from the precipitations with ethanol and ammonium sulphate which are used to extract the enzyme from the tissue. The present invention not only overcomes many of these problems associated with the conventional method for producing peroxidase, it also produces a new peroxidase with improved properties in relatively high yields.

Various other sources have been suggested in the literature for the commercial production of peroxidase. For example, radish plant cell cultures have been suggested as a source of extracellular peroxidase (Plant Cell, Tissue and Organ Culture, 1989, 18:321-327) but the yields and specific activity of the product enzyme are low.

A plant cell culture technique for the production HRPO is disclosed in J. Fermentation Technology, 1989, 67:31-34 based on cultures established from genetically modified horseradish cells, modified to increase the stability of the enzyme.

It has also been suggested that calli cultured from various plant species can be used as a source for the production of peroxidase, but callus culture is inherently incapable of large scale commercial production. Such suggestions are contained in unexamined published Japanese patent applications:

JP-A-1222778 (source species:

Glycyrrhiza glabra L.var,
Ipomoea batatas Lam. var deulis Makino,
Stevia rebaudiana Bertoni, and
Bupleurum falcatum L.)

JP-A-1222777 (source species:

Zoysia japonica, and
Zoysia macrostachchya)

JP-A-1222776 (source species:

Trifolium repens L.,
Carica papaya L.,
Phellodendron amurense Rupr.,
Oenothera lamarchiana Ser.,
Scopolia japonica Maxim,
Lithospermum erythrorhizon Sieb et Zucc.,
Glycine max Merrill, and
Gynastema pentaphyllum Makino.)

JP-A-63233782 (source species: general) JP-A-62138188 (source species: Ipomoea aquatica Forsk.)

Amongst other plants known to produce peroxidase are plants of the genus <u>Acer</u> (sycamore), although, so far as the present applicants are aware, nobody has yet proposed to produce peroxidase commercially from <u>Acer</u>, and certainly not by means of a cell suspension culture. Thus, J. de Microscopie, 1970, 9:1089-1102 reports on the microscopic examination of sycamore leaf tissue and cultured cell sections of sycamore tissue which have been stained using a chromogenic peroxidase substrate to show up the microscopic intra-cellular structures.

In a paper presented to and published in the proceedings of the Fourth European Congress of Biotechnology, 1987, 2:342-344, the present inventors reported the results of a comparative study on the effect of peroxidase in plant cell cultures from four different species including inter alia <u>Acer,</u> with specific reference to the production of intra-cellular peroxidase and its involvement in the degradation of secondary products of the cell suspension culture, but did not identify suspension cultures of <u>Acer</u> as a potential commercial source of a high activity, high yield extra-cellular peroxidase.

In Physiologia Plantarum, 1970, 23:1212-1222 the authors report a study of intra-cellular peroxidase activity in sycamore cells conducted with a view to establishing various metabolic pathways in the plant metabolism, but with no suggestion as to any commercial utility or even any extra-cellular peroxidase activity.

Finally the production of an intra-cellular glutathione peroxidase in sycamore cells has been reported in Plant Science, 1985, 42: 35-40.

In contrast to the foregoing, it has now been found that a highly active, highly stable peroxidase enzyme is produced extra-cellularly in high yield by suspension plant cell cultures of plant cells from plants of the genus <u>Acer,</u> and especially plant cell cultures of <u>Acer</u> <u>pseudoplatanus,</u> and especially root cell cultures. The fact that the peroxidase is produced extra-cellularly in high yield (with approximately 80% of the total peroxidase being produced extra-cellularly) is highly surprising, and greatly simplifies product recovery. Indeed for many commercial purposes, it is sufficient merely to separate the supernatant liquor containing the extra-cellular peroxidase from the culture, either batchwise or continuously, to provide a crude peroxidase containing liquor which can be used as such, or which can be further concentrated, if needed, e.g. by evaporation, freeze drying or ultrafiltration. However, if a substantially pure peroxidase is required this can be recovered from the culture liquor by simple separation means, such as chromatography. Either way the risk of deactivation of the enzyme during the recovery and purification procedures is substantially reduced. Also, since such a high proportion of the enzyme is produced extra-cellularly, waste disposal problems are simplified since the volume of cellular tissue remaining at the end of the culture and recovery of the peroxidase is relatively low. If desired, that cellular tissue can be further processed, after completion of the culture and separation of the cultured cells from the supernatant liquor, to recover intra-cellular peroxidase as an additional product, thus contributing considerably to the over all economies of the process. That intra-cellular peroxidase may be recovered, for example, by the established procedures of disrupting the cells to release the intra-cellular peroxidase followed by extraction and precipitation with ethanol and ammonium sulphate in the conventional way.

In accordance with a first aspect, therefore, of the present invention, there is provided a process for the production of peroxidase enzyme which comprises establishing a plant cell culture of extra-cellular peroxidase producing cells from a plant of the genus <u>Acer</u> in suspension in a culture medium capable of supporting the growth of the extra-cellular peroxidase producing cells, continuing the culture of said cells in suspension in the culture medium with the concomitant accumulation of extra-cellular peroxidase in the culture medium, and recovering the accumulated peroxidase from the culture medium.

The resulting extra-cellular peroxidase has advantageous properties over the conventional horseradish peroxidase. Included among these properties are a high initial level of activity and an improved thermal stability at high temperatures. This is very useful in some industries where a reaction may be more effective at a higher working temperature but is unable to operate there due to the very sensitive nature of enzymes which denature under adverse thermal conditions. Additionally, the peroxidase according to this invention has a high storage stability.

This invention thus provides in substantially pure form peroxidase enzyme having the following characteristics:

| Source | Acer pseudoplatanus |
|---|---|
| Location | Extra-cellular |
| Mol.wt.(Gel.Filtration) | Approx. 37000 |
| pH range | 4 - 9 |
| Optimum pH | 5.5 |
| Optimum assay temperature | 45°C |
| Stability | Retains at least 50% of optimum activity at 65 to 75°C. |
| Reactivity | Substantially $H_2O_2$ specific, but reactive in the presence of: guaiacol, pyrogallol, aminophenol or O-dianisidine as the reductant. |

(continued)

| Source | Acer pseudoplatanus |
|---|---|
| Elution profile | Six distinct activity peaks - see Figure 4. |

The process of the present invention begins with the initiation and maintenance of a suspension culture of the extra-cellular peroxidase producing Acer plant cells, preferably Acer pseudoplatanus. This is best achieved by germinating the Acer seeds, preferably seeds of Acer pseudoplatanus on a suitable growth medium, e.g. agar, excising root tissue from the germinated seeds, transferring the excised root tissue on to fresh growth medium and continuing the growth of the root tissue in said fresh medium to produce root tissue calli, which can then be comminuted and transferred into a liquid growth medium to establish a suspension culture of the root tissue. This initial culture can then be subcultured to provide for the large scale production and accumulation of extra-cellular peroxidase in the culture medium.

As the culture media for the callus and suspension cultures there may be used any suitable culture medium for plant cell cultures, e.g. Gamborgs B5 medium or Murashige and Skoog (MS) medium containing a suitable carbon source, e.g. one or more of glucose, sucrose and fructose in an amount of from 1-15% by weight, preferably from about 2-5%, and most preferably about 3%, and the necessary phytohormones, e.g. dichlorophenoxyacetic acid and kinetin. For the callus culture Gamborgs B5 medium is preferably used. For the suspension (production) culture the preferred medium is the MS medium.

A characteristic feature of the plant cell cultures used in accordance with the present invention, particularly the root cell cultures of Acer Pseudoplatanus is that it has been found possible to use carbohydrate waste materials containing glucose and/or sucrose, such as are produced in large amounts in the confectionery industry, as the carbon source in the culture medium, rather than the customarily used refined glucose and/or sucrose. Glucose and/or sucrose containing wastes produced in the confectionery industry, e.g. floor sweepings, mis-shapen moulded confectioneries and discontinued lines, present a substantial disposal problem in the confectionery industry since it is usually not economic to reprocess the waste, and simply dumping the waste presents environmental problems. In accordance with the present invention, it has been found that such wastes provide an inexpensive source of glucose and/or sucrose for the plant cell cultures and which can be supplied simply by extracting the waste confectionery with water and feeding the crude extract, without further purification or refinement, to the culture medium, the plant cell culture being remarkably tolerant to the other water soluble ingredients, dyes etc. in the waste confectionery.

The necessary phytohormones used in the medium are 2,4-dichlorophenoxyacetic acid at around lmg. $L^{-1}$ and 6-furfurylaminopurine (kinetin) at around 0.1 mg. $L^{-1}$.

Aerobic culture conditions are used preferably at a pH from 5.6 to 5.8 and at a temperature of about 25°C, with optimum peroxidase activity occurring in the culture medium after about 8 days of continuous culture.

A preferred method according to this invention will now be described in detail with reference to the production of extra-cellular peroxidase from Acer pseudoplatanus cell cultures, and with reference to the accompanying drawings, in which:

Fig.1 shows the growth profile of A. pseudoplatanus root cell cultures in MS medium supplie with 3% sucrose/glucose in a 30L airlift fermentor.

Fig.2 shows the extra-cellular peroxidase production profile of A. pseudoplatanus root cell culture in MS medium supplied with 3% sucrose/glucose in a 250 mL Erlenmeyer flask.

Fig.3 shows the thermal stability profile of the A. pseudoplatanus extra-cellular peroxidase.

Fig.4 shows the elution profile.

Initiation and maintenance of the cell culture

Sycamore cell cultures are initiated from root tissue of Acer pseudoplatanus by first germinating seeds of A. pseudoplatanus on agar medium. The seeds are first sterilised by immersion in 15% hypochlorite solution with shaking on a gyrotatory shaker for approximately 40 minutes at 24°C. After this sterilisation, the seeds are washed with copious amounts of sterile distilled water to remove all traces of hypochlorite. The seeds are then placed on agar and incubated at 25°C. After germination, small segments of root are excised aseptically and transferred onto fresh agar medium. Periodically the tissue is examined for callus formation. Callus cultures are then initiated and established by routine subculture, following which portions of the callus culture tissue are transferred into a liquid culture medium to establish the cell suspension culture. During the establishment of the suspension culture viability tests are carried out on the cell lines using the fluorescin diacetate method (described below). In this way the suspension cultures can be subcultured by volume on a fortnightly basis, i.e. they can be maintained routinely by the aseptic transfer of 20 mL amounts of 14 day old cultures into 100 mL aliquots of sterilised culture medium in 250 ml Erlenmeyer flasks. The flasks are placed on a gyrotatory shaker at 150 rpm in the light at 25°C.

The cell line is initiated on B5 medium (Gamborg et al., Exp.Cell Res.50, 148-151, 1968) with 3% sucrose as the carbon source, but is later transferred on to Murashige & Skoog (MS) medium (Murashige et al. Physiol. Plant 15, 473-497, 1962) with 3% glucose as the carbon source. The phytohormones added to the medium are 1 mg.L$^{-1}$ 2,4-dichlorophenoxyacetic acid and 0.1 mg.L$^{-1}$ 6-furfurylaminopurine (kinetin). The pH of the growth medium is adjusted to 5.6 - 5.8 prior to sterilisation.

Sycamore culture details

For small scale experimental work the cultures are subcultured by weight, i.e. 3 g fresh weight per 100 ml medium. For intermediate scale experiments (up to 10 L), two litres of 14 day old culture can be used as inoculum in 8L of fresh medium in a reaction vessel equipped with a stirrer. Suitable intermediate scale culture conditions include pH 5.6 to 5.8 temperature 25°C and a compressed air feed of about 1Lmin$^{-1}$.

Large scale cultures can be conducted in similar manner in an LH (Registered TM) 30 L airlift fermentor, for example by inoculation with 14 day old root cell cultures sufficient to give a fresh weight of 20g.L$^{-1}$ (approximately 2.0 g.L$^{-1}$ dry weight). The vessel is aerated with compressed air at a rate of 6L.min$^{-1}$ and at a temperature of 25°C.

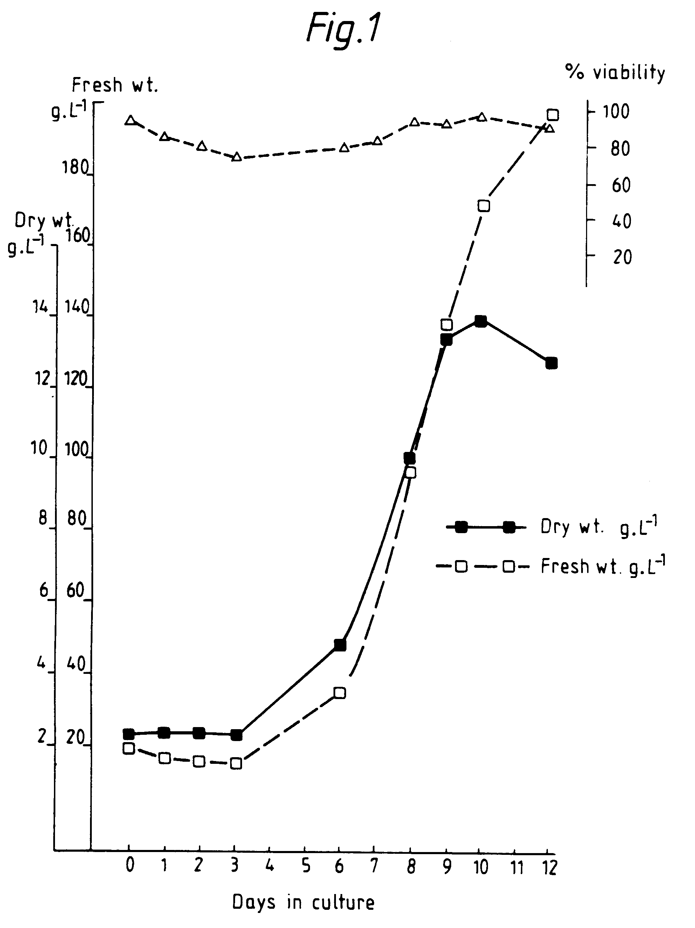

Figure 1 of the accompanying drawings shows a growth profile for a sycamore culture grown in a 30 L airlift fermentor. This shows an optimum cell viability after 9 days in the fermentor, an optimum cell fresh weight after 11 days in the fermentor and an optimum cell dry weight after 10 days in the fermentor.

Experimental methods

By aseptically taking small culture samples from the culture vessel at regular intervals, and monitoring fresh and dry weight measurements, cell viability, culture pH and peroxidase activity, it is possible to determine an optimum culture time and optimum conditions for the efficient production of peroxidase. These experiments are detailed below:

Fresh and dry weight measurements

A Whatman (Registered Trade Mark) No. 1 filter paper disc, 2.5 cm in diameter, is weighed directly after its removal from an oven where it has been drying at 60°C for at least 24 hours. The disc i then placed on a stainless steel filter bed seated on a rubber stopper in a Buchner flask. The disc is wetted with distilled water and a vacuum applied to the flask for approximately 10 seconds to remove excess water. The wet filter disc is then weighed immediately. The disc was replaced on the filter bed and the filter top is positioned directly over the disc. A 3 mL portion of culture is filtered through the system and a vacuum applied to remove any excess of medium. The disc with the cells thereon is weighed immediately and placed in an oven at 60°C for at least 24 hours before being reweighed. Calculations of the fresh and dry weights of the cultures are made using the following equations:

$$\text{Wet weight (g/L)} = \left\{ \frac{\underline{\text{Wt of wet (disc + cells)} - \text{wt of wet disc}}}{\text{Sample volume (mL)}} \right\} \times 1000$$

$$\text{Dry weight (g/L)} = \left\{ \frac{\underline{\text{Wt of dry (disc + cells)} - \text{wt of dry disc}}}{\text{Sample volume (mL)}} \right\} \times 1000$$

Viability tests

One drop of culture together with one drop of fluorescin diacetate solution (1:50 dilution with water of a stock solution made up of 5 mg fluorescin diacetate in 1 ml acetone) is placed on a cavity microscope slide and a cover slip placed over it. After approximately 2 minutes the resulting fluorescence is observed using a fluorescence microscope equipped with an exciter filter. Viable cells exhibit a bright green fluorescence. Culture viability is estimated by counting the number of cells that fluoresce in a given field and expressing this as a percentage of the total number of cells in that field.

## Estimation of Peroxidase activity

Firstly the samples from the culture are fractionated into cell and medium fractions in order to determine the peroxidase activity in each.

The cells are disrupted on ice using a pre-chilled mortar and pestle with approximately 10% (w/w) pre-chilled, acid-washed sand and ice cold 0.1 M McIlvaine's citric acid phosphate buffer* pH 5.5. An Eppendorf system was used to centrifuge the cell homogenate for 2 minutes to remove the sand and debris. The resulting supernatant was transferred to clean tubes and centrifuged for a further 5 minutes. The supernatant was carefully removed from the centrifuge tube and kept on ice before use.

## Peroxidase assay

Peroxidase catalyses a host of reaction in which hydrogen peroxide is a specific oxidising agent and a wide range of substrates act as electron donors.

$$H_2O_2 + XH \text{-------} > XOH + H_2O$$

The electron donor used in the assay described in this application is guaiacol (Bergmeyer, 1983).

$$H_2O_2 + 4 \text{ guaiacol} \text{-------} > H_2O + \text{tetra-guaiacal}$$

In this case the assay mixture is prepared as follows:

|  | Assay Mixture | |
|---|---|---|
|  | For Medium Enzyme | For Cell Enzyme |
| 0.1M McIlvaine's citric acid phosphate buffer, pH 5.5 | 2.80 mL | 2.84 mL |
| 0.15 M guaiacol (18.3 g.L $^{-1}$) | 0.10 mL | 0.10 mL |
| 43.7 mM $H_2O_2$ | 0.04 mL | 0.04 mL |
| Cell extract | - | 0.02 mL |
| Medium | 0.06 mL | - |

The reaction is started by the addition of hydrogen peroxide. Although the volume of cell extract is altered depending upon the activity of the enzyme, however the total assay volume is maintained at 3.0 ml by a corresponding change in the volume of buffer used. The appearance of tetra-guaiacal, i.e. an increase in absorbence at 436 nm, is monitored during the reaction at 25°C.

Peroxidase activity (units/mL) in the cell extract and the medium is calculated according to the formula:

$$\text{Activity Units/mL} = \frac{\Delta/\text{min} \times 4 \times \text{reaction volume (mL)}}{25.5 \times \text{sample volume (ml)}}$$

One unit of enzyme activity is defined as μmoles of substrate utilised per minute. Where $\Delta/\text{min}$ is the change in absorbence per minute at 436 nm, and the constant 25.5 is the molar extinction coefficient (cm$^2$/mole) for tetra-guaiacal.

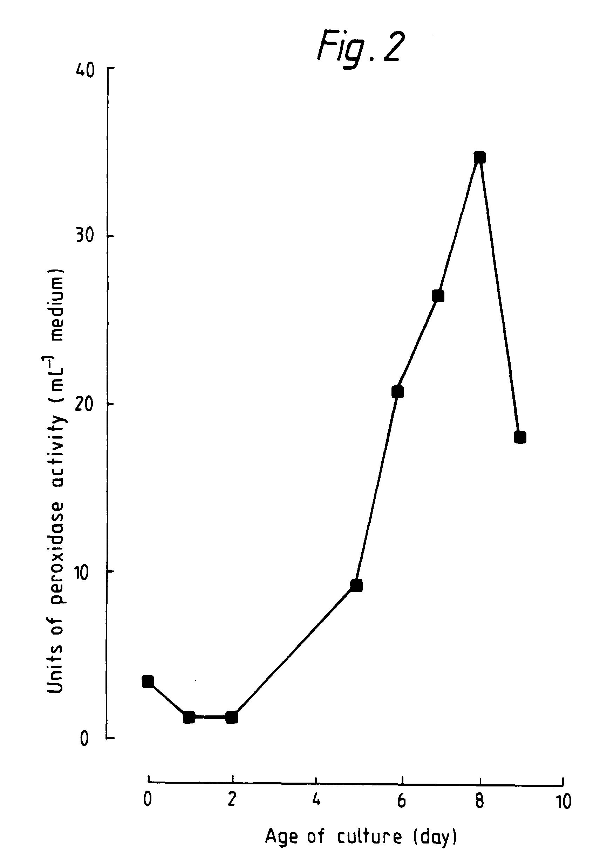

Figure 2 of the accompanying drawings represents a graph showing the peroxidase production profile in the medium from a suspension root cell culture of Acer psuedoplatanus in 250 mL Erlenmeyer flasks. On day 8 of culture there is a substantial increase in medium peroxidase activity to reach 34,500 units per litre of medium. This level of activity corresponds to approximately 80% of total peroxidase activity in the culture. Thus it would appear that day 8 of the culture is optimum for the extraction of peroxidase.

Extraction of peroxidase from the culture is achieved by conventional downstream processing techniques, and

(* McIlvaine's citric acid phosphate buffer: 43.10ml 0.1M citric acid and 56.90 ml 0.2M disodium phosphate).

here the advantage of this process lies in the fact that approximately 80% of the peroxidase is present in the culture medium and therefore is relatively easily extracted. After separating the cells from the medium, the latter is concentrated using ultrafiltration by first passing the liquor through a 100,000 molecular weight cut-off filter, followed by a 20,000 molecular weight cut-off filter. For high purity enzyme conventional chromatography techniques can be employed.

The characteristics of the peroxidase product obtained are as follows:

Molecular weight (gel filtration): about 37000;

| pH range | 4 to 9; |
|---|---|
| Optimum pH | 5.5; |
| Optimum assay | 45°C |

Temperature stability:

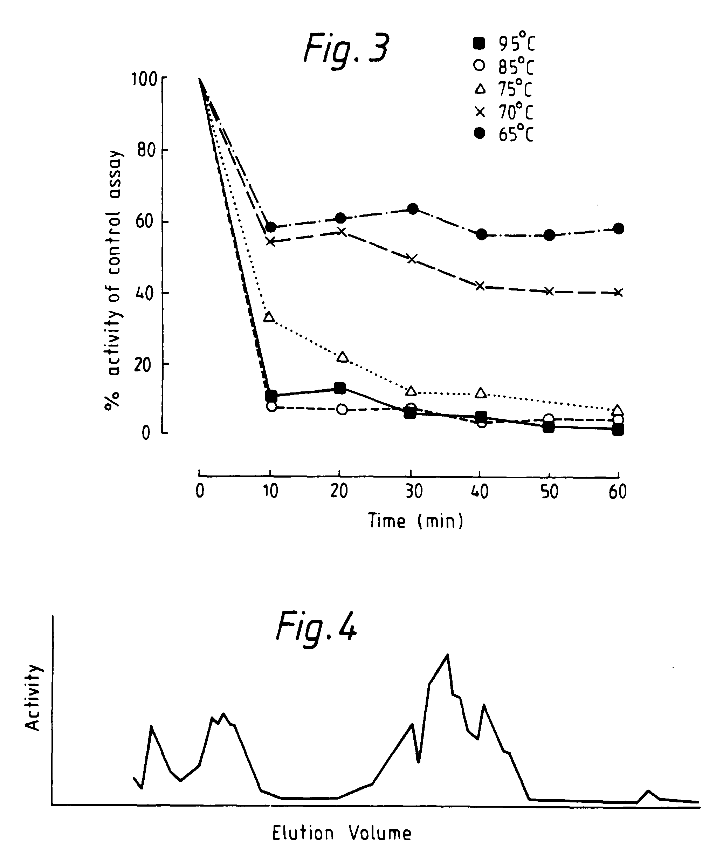

As shown by accompanying Fig.3, the peroxidase retains approximately 50% of its original activity when heated at 65 to 70°C for one hour, after an initial decrease in activity over the first 10 minutes. Other tests have shown that some activity is retained even after heating to 95°C.

Storage stability:

The peroxidase has been shown to retain its activity for prolonged periods of time, e.g. 12 months or more at temperatures of from +4°C to -20°C. The enzyme can be stored in crude form, as a suspension in ammonium sulphate or freeze dried.

Elution Profile:

When eluted from a Whatman CM52 ion-exchange column using a gradient of 0.01 to 1.0M sodium acetate buffer at pH 5.0, a characteristic 6- peak profile is obtained, see Fig.4.

## Claims

1. A process for the production of peroxidase enzyme which comprises establishing a plant cell culture of extra-cellular peroxidase producing cells from a plant of the genus Acer in suspension in a culture medium capable of supporting the growth of the extra-cellular peroxidase producing cells, continuing the culture of said cells in suspension in the culture medium with the concomitant accumulation of extra-cellular peroxidase in the culture medium, and recovering the accumulated peroxidase from the culture medium.

2. A process according to claim 1, wherein the extra-cellular peroxidase producing plant cells are cells from the species Acer pseudoplatanus.

3. A process according to claim 1 or 2, wherein the extra-cellular peroxidase producing plant cells are root cells.

4. A process according to claim 3, wherein the suspension culture is established from germinated plant seeds by first germinating the plant seeds on a first growth medium, transferring sections of root tissue from the germinated seeds to fresh growth medium, allowing the transferred root tissue to form a callus, disrupting the callus and dispersing the disrupted callus cells into the culture medium to form a suspension culture.

5. A process according to any one of claims 1 - 4, where in the culture medium used in the suspension culture of the extra-cellular peroxidase producing plant cells contains as a carbon source from 1-15% by weight fructose, glucose or sucrose.

6. A process according to claim 5, wherein there is used as the carbon-containing source a carbohydrate waste material containing glucose and/or sucrose.

7. A process according to claim 6, wherein the glucose and/or sucrose containing waste is a confectionery waste.

8. A process according to any one of claims 2 - 8, wherein the cells are cultured in the suspension culture under aerobic conditions at a temperature of about 25°C and at a pH in the range 5.6 to 5.8.

9. A process according to any one of claims 1 - 8, wherein the product extra-cellular peroxidase is recovered from the culture as a crude extract by separating the supernatant peroxidase-containing liquor from the culture and concentrating the liquor to produce a crude peroxidase containing concentrate.

10. A process according to any one of claims 1 - 9, wherein following the suspension culture, the cultured plant cell tissue is separated from the culture medium to provide a supernatant liquor containing the accumulated extra-cellular peroxidase, recovering the extra-cellular peroxidase from the supernatant liquor and further processing the separated plant cell tissue to recover intra-cellular peroxidase as a separate product.

11. A process according to claim 10, wherein the intra-cellular peroxidase is recovered from the separated plant cell tissue by disrupting the cells in the separated plant cell tissue, extracting the intra-cellular peroxidase from the disrupted plant cells by extraction with ethanol and precipitating the extracted intra-cellular peroxidase with ammonium sulphate.

**Patentansprüche**

1. Verfahren zur Hertellung von Peroxidase-Enzym, bei dem man eine Pflanzenzellkultur extrazelluläre Peroxidase produzierender Zellen aus einer Planze der Gattung *Acer* in Suspension in einem Kulturmedium, das das Wachstum der extrazelluläre Peroxidase produzierenden Zellen unterstützen kann, erzeugt, das Züchten dieser Zellen in Suspension in dem Kulturmedium mit der gleichzeitigen Ansammlung extrazellulärer Peroxidase in dem Kulturmedium fortsetzt und die angesammelte Peroxidase aus dem Kulturmedium gewinnt.

2. Verfahren nach Anspruch 1, bei dem die extrazelluläre Peroxidase produzierenden Pflanzenzellen Zellen der Art *Acer pseudoplatanus* sind.

3. Verfahren nach Anspruch 1 oder 2, bei dem die extrazelluläre Peroxidase erzeugenden Pflanzenzellen Wurzelzellen sind.

4. Verfahren nach Anspruch 3, bei dem die Suspensionskultur aus gekeimten Pflanzensamen erzeugt wird, indem man zunächst die Pflanzensamen auf einem ersten Wachstumsmedium keimt, Bereiche von Wurzelgewebe von den gekeimten Samen auf frisches Wachstumsmedium überführt, das überführte Wurzelgewebe einen Kallus bilden läßt, den Kallus zertrennt und die zertrennten Kalluszellen in dem Kulturmedium unter Bildung einer Suspensionskultur dispergiert.

5. Verfahren nach einem der Ansprüche 1 bis 4, bei dem das in der Suspensionskultur der extrazelluläre Peroxidase produzierenden Pflanzenzellen verwendete Kulturmedium als eine Kohlenstoffquelle 1 bis 15 Gew.% Fructose, Glucose oder Saccharose enthält.

6. Verfahren nach Anspruch 5, bei dem als die kohlenstoffhaltige Quelle ein Glucose und/oder Saccharose enthaltendes Kohlenhydrat-Abfallmaterial verwendet wird.

7. Verfahren nach Anspruch 6, bei dem das Glucose und/oder Saccharose enthaltende Abfallmaterial ein Süßwarenabfall ist.

8. Verfahren nach einem der Ansprüche 2 bis 8, bei dem die Zellen in der Suspensionskultur unter aeroben Bedingungen bei einer Temperatur von etwa 25 °C und einem pH-Wert im Bereich von 5,6 bis 5,8 gezüchtet werden.

9. Verfahren nach einem der Ansprüche 1 bis 8, bei dem das extrazelluläre Peroxidaseprodukt aus der Kultur als ein Rohextrakt gewonnen wird, indem man die obenschwimmende peroxidasehaltige Flüssigkeit von der Kultur abtrennt und die Flüssigkeit unter Bildung eines Rohperoxidase enthaltenden Konzentrates konzentriert.

10. Verfahren nach einem der Ansprüche 1 bis 9, bei dem nach dem Züchten in Suspension das gezüchtete Pflanzenzellengewebe von dem Kulturmedium abgetrennt wird, um eine die angesammelte extrazelluläre Peroxidase enthaltende obenschwimmende Flüssigkeit zu bekommen, wobei man die extrazelluläre Peroxidase aus der obenschwimmenden Flüssigkeit gewinnt und das abgetrennte Pflanzenzellengewebe weiter bearbeitet, um intrazelluläre Peroxidase als ein getrenntes Produkt zu gewinnen.

11. Verfahren nach Anspruch 10, bei dem die intrazelluläre Peroxidase aus dem abgetrennten Pflanzenzellengewebe gewonnen wird, indem man die Zellen in dem abgetrennten Pflanzenzellengewebe zertrennt, die intrazelluläre Peroxidase aus den zertrennten Pflanzenzellen durch Extraktion mit Ethanol extrahiert und die extrahierte intrazelluläre Peroxidase mit Ammoniumsulfat ausfällt.

**Revendications**

1. Procédé de production d'une enzyme peroxydase, comprenant les étapes consistant à établir une culture de cellules végétales formée de cellules produisant une peroxydase extracellulaire, provenant d'une plante du genre *Acer,* en suspension dans un milieu de culture capable de soutenir la croissance des cellules produisant une peroxydase extracellulaire, continuer la culture desdites cellules en suspension dans le milieu de culture avec accumulation concomitante de peroxydase extracellulaire dans le milieu de culture et recueillir la peroxydase accumulée à partir du milieu de culture.

2. Procédé selon la revendication 1, dans lequel les cellules végétales produisant une peroxydase extracellulaire sont des cellules provenant de l'espèce *Acer pseudoplatanus.*

3. Procédé selon la revendication 1 ou 2, dans lequel les cellules végétales produisant une peroxydase extracellulaire sont des cellules provenant de racines.

4. Procédé selon la revendication 3, dans lequel on établit la culture en suspension à partir de graines végétales germées, d'abord en faisant germer les graines végétales sur un premier milieu de croissance, en transférant des coupes de tissu de racines provenant des graines germées à un milieu de croissance frais, en permettant au tissu de racines transféré de former un cal, en rompant le cal et en dispersant les cellules du cal rompu dans le milieu de culture pour former une culture en suspension.

5. Procédé selon l'une quelconque des revendications 1 à 4, dans lequel le milieu de culture utilisé dans la culture en suspension des cellules végétales produisant une peroxydase extracellulaire contient de 1 à 15 % en poids de fructose, de glucose ou de saccharose comme source de carbone.

6. Procédé selon la revendication 5, dans lequel on utilise, comme source contenant du carbone, un déchet glucidique contenant du glucose et/ou du saccharose.

7. Procédé selon la revendication 6, dans lequel le déchet contenant du glucose et/ou du saccharose est un déchet de confiserie.

8. Procédé selon l'une quelconque des revendications 1 à 7, dans lequel on cultive les cellules dans la culture en suspension dans des conditions aérobie à une température d'environ 25°C et à un pH compris entre 5,6 et 5,8.

9. Procédé selon l'une quelconque des revendications 1 à 8, dans lequel on recueille le produit peroxydase extracellulaire à partir de la culture sous la forme d'un extrait brut en séparant le liquide surnageant contenant la peroxydase de la culture et en concentrant le liquide pour produire un concentré brut contenant la peroxydase.

10. Procédé selon l'une quelconque des revendications 1 à 9, dans lequel, après la culture en suspension, on sépare le tissu de cellules végétales cultivées du milieu de culture pour fournir un liquide surnageant contenant la peroxydase extracellulaire accumulée, on recueille la peroxydase extracellulaire à partir du liquide surnageant et on retraite ensuite le tissu de cellules végétales séparé pour recueillir la peroxydase intracellulaire sous la forme d'un produit séparé.

11. Procédé selon la revendication 10, dans lequel on recueille la peroxydase intracellulaire à partir du tissu de cellules végétales séparé en rompant les cellules dans le tissu de cellules végétales séparé, en extrayant la peroxydase intracellulaire des cellules végétales rompues par extraction avec de l'éthanol et précipitation de la peroxydase intracellulaire extraite dans du sulfate d'ammonium.

# Fig.1

# Fig. 2

Fig. 3

■ 95°C
○ 85°C
△ 75°C
× 70°C
● 65°C

% activity of control assay

Time (min)

Fig. 4

Activity

Elution Volume